(19) Europäisches Patentamt
European Patent Office
Office européen des brevets

(11) EP 4 134 373 A1

(12) EUROPEAN PATENT APPLICATION

(43) Date of publication:
**15.02.2023 Bulletin 2023/07**

(21) Application number: **21191143.3**

(22) Date of filing: **12.08.2021**

(51) International Patent Classification (IPC):
***C07J 71/00*** *(1974.07)* ***C07J 5/00*** *(1974.07)*
***A61P 5/44*** *(2000.01)* ***A61K 31/573*** *(2000.01)*

(52) Cooperative Patent Classification (CPC):
**C07J 71/0015; A61P 5/44; C07J 5/0076**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Santhera Pharmaceuticals (Schweiz) AG**
**4133 Pratteln (CH)**

(72) Inventors:
- **Schäfer, Gabriel**
  **4123 Allschwil (CH)**
- **Olivieri, Lauso**
  **00072 Ariccia (IT)**
- **Martin, Marco**
  **35031 Abano Terme (IT)**

(74) Representative: **Grünecker Patent- und Rechtsanwälte PartG mbB**
**Leopoldstraße 4**
**80802 München (DE)**

# (54) SYNTHESIS OF DELTA 9,11 STEROIDS

(57) The present invention is directed to a process for the preparation of Δ9,11 steroids by deoxygenation of 9,11-epoxy steroids using HI. The disclosed process selectively forms Δ9,11 steroids such as Vamorolone and can also be used for steroid synthesis of Δ9,11 steroids in pharmaceutical purity.

EP 4 134 373 A1

## Description

Described is a process for the preparation of Δ9,11 steroids by deoxygenation of 9,11-epoxy steroids using HI. The disclosed process selectively forms Δ9,11 steroids such as Vamorolone and can also be used for steroid synthesis of Δ9,11 steroids in pharmaceutical purity.

BACKGROUND OF THE INVENTION

Glucocorticoids are standard of care for many inflammatory conditions, but longterm use is associated with a broad array of side effects. Recently, it was found that Δ9,11 steroid analogs hold promise as a source of safer agents for treating chronic inflammatory disorders.

Typical glucocorticoids like cortisol, prednisone and prednisolone have Δ9,11 steroid counterparts, such as Tirilazad, VBP1 and Anecortave. For such Δ9,11 steroids new syntheses are of need.

Furthermore, the synthesis of corticosteroids having therapeutic utility, such as mometasone, metametasone and beclometasone, requires functionalization of the C-9 and C-11 positions of the steroid molecule. The functionality is generally introduced via Δ9,11 steroid intermediates. The synthesis of Δ9,11 steroids having therapeutic utility, and the synthesis of Δ9,11 steroid intermediates is, thus, of growing importance.

Methods for preparing Δ9,11 steroids, i.e. steroids having a 9,11-double bond, are known in the art. For example, an 11-hydroxy steroid can be converted to the corresponding mesylate (by treating the steroid with mesyl chloride) which is transformed into a Δ9,11 steroid via an elimination reaction. However, the respective prior art methods are not regiospecific in the case of starting from 11-hydroxy steroids and typically lead to mixtures of Δ9,11 steroid containing high and unwanted amounts of Δ11,12 steroids. Separation of these regio-isomeric products is difficult, generally requiring laborious physical separation procedures, resulting in increased costs and lower yields.

Other synthesis strategies are known as well: US 5,399,727 discloses the synthesis of a specific Δ9,11 steroid from a 9-hydroxy steroid with chlorosulfonic acid in an organic solvent. EP 0 969 012 discloses a process for the regioselective dehydration of 11-hydroxy steroids using $PCl_5$, $PCl_3$, $POCl_3$ or either $SO_2Cl_2$ and imidazole, or $PPh_3$ and $CCl_4$. These processes are quite harsh, require toxic chemicals and lead to unintended side-products and impurities. Vamorolone (17α,21-Dihydroxy-16α-methylpregna-1,4,9(11)-triene-3,20-dione) is an Active Pharmaceutical Ingredient (API), which is a clinical candidate, e.g. for treating DMD. Vamorolone is, thus, a new Δ9,11 steroid of high therapeutic interest.

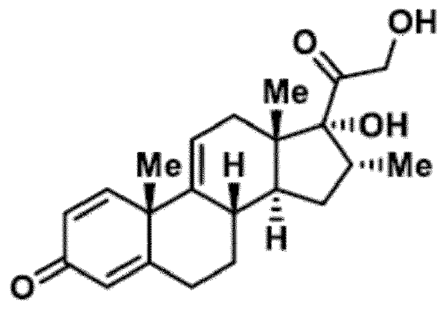

*Vamorolone*

Vamorolone is currently produced from the commercially available 3TR (Tetraene acetate) - see Scheme 2. In step a, TMS imidazole, MeMgCl and THF are added to 3TR, with subsequent addition of $CuAc_2$, $H_2O$, DMPU, MeMgCl and THF in step b. Under treatment with peracetic acid in Toluene from compound 2 the intermediate 3 is formed in step c. After treatment with $NaHSO_3$ and TFA (step d), EtOAc and heptane (step e) and acetonitrile trituration (step f) HBr in $CH_2Cl_2$ is added (step g) and MeOH (step h) is used for crystallization to form Acetyl-Vamorolone 4. Acetyl-Vamorolone is deacetylated with $K_2CO_3$ in MeOH, followed by HCl to obtain Vamorolone (step i). The synthesis is disclosed in Bioorganic & Medicinal Chemistry, Volume 21, Issue 8, 15 April 2013, Pages 2241-2249.

*Scheme 2: Vamorolone preparation in the prior art*

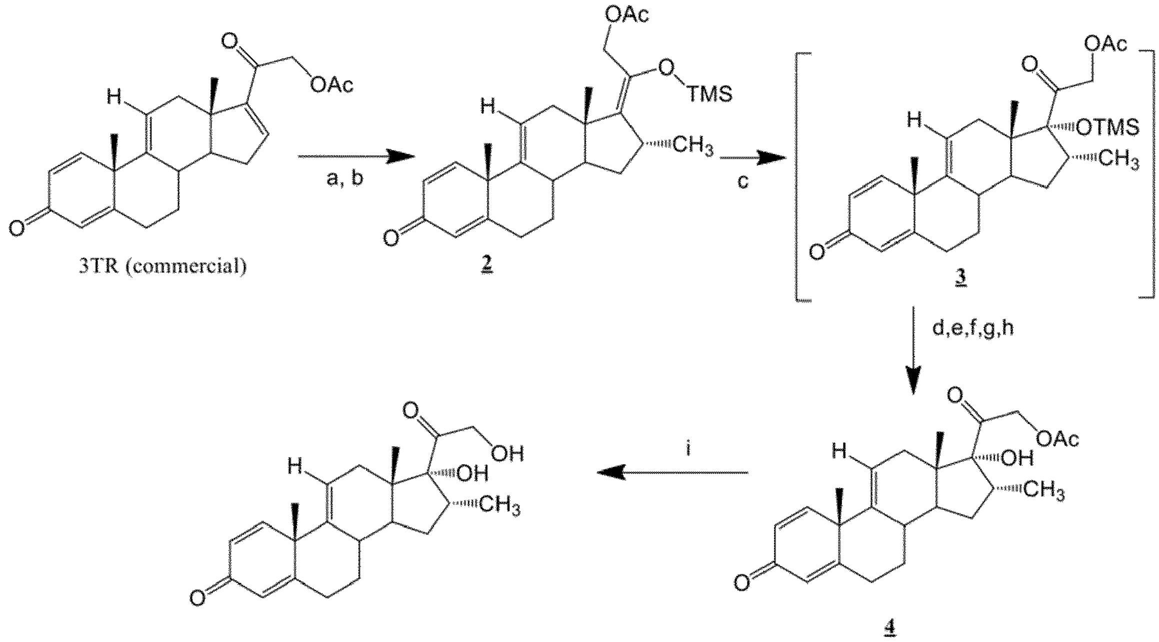


This process is difficult to control, requires the use of several toxic and hazardous chemicals and thus represents an unattractive synthesis of Vamorolone.

Overall, there is a need for a more straight forward and safe way of producing Vamorolone and other Δ9,11 steroids in high yields, and in controlled pharmaceutical purity eliminating the presence of numerous impurities and side-products (e.g. steroid-side-products) in the final product.

## SUMMARY OF THE INVENTION

The present invention fulfils this need by providing a convenient, non-toxic and quantitative synthesis of Vamorolone and other Δ9,11 steroids. The new synthesis is surprisingly simple and significantly reduces steroid-side-products. For the synthesis of Vamorolone and other Δ9,11 steroids HI is used to deoxygenate 9,11 epoxide steroid precursors, such as the protected, e.g. acetylated, commercial 8-DM or the unprotected commercial 8-DM ((1*S*,2*S*,13*R*,14*R*,15*S*,17*S*)-14-hydroxy-14-(2-hydroxyacetyl)-2, 13, 15-trimethyl-18-oxapenta-cyclo[8.8.0.$0^{1,17}$.$0^{2,7}$.$0^{11,15}$]octadeca-3,6-dien-5-one) Δ9,11 steroids, such as 8-DM, are commercially available and protection groups (to be attached to the 21-OH moiety) can be attached using conventional synthesis means available in the art.

Accordingly, the present invention provides a process for preparing a Δ9,11 steroid of the formula I,

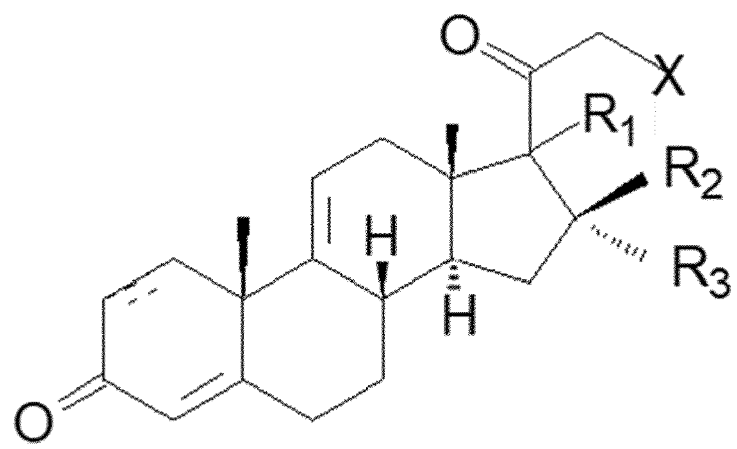

I

wherein the dotted line is a single or a double bond; $R^1$ is H or OH; one of $R^2$ or $R^3$ is $CH_3$ and the other is H or both are H; and X is H, halo, OR, wherein R is H or C(O)$R^1$, and R' is $CF_3$, $C_1$-$C_6$ alkyl, $C_5$-$C_{12}$ cycloalkyl, aryl, heteroaryl or $C_1$-$C_6$ alkoxy, or NR", wherein $NR^{II}$ is a tertiary amine; the tertiary amine throughout the present specification may be part of a linear or cyclic substructure.

Preferred is a process for preparing a Δ9,11 steroid of the formula Ia,

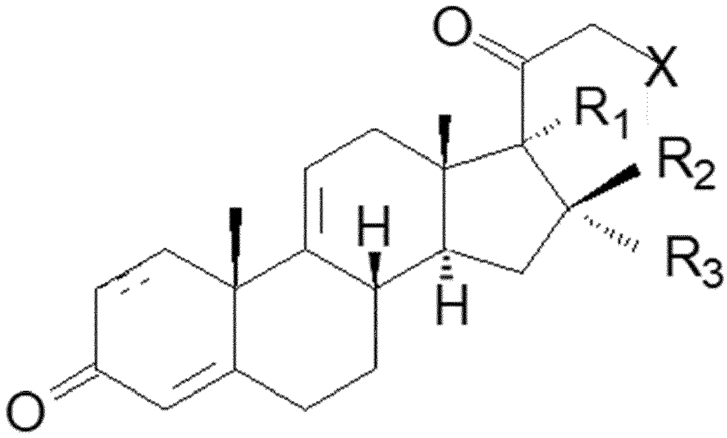

Ia

wherein:

The dotted line is a single or a double bond; $R^1$ is H or OH; one of $R^2$ or $R^3$ is $CH_3$ and the other is H or both are H; and X is H, halo, OR, wherein R is H or C(O)$R^I$, and $R^I$ is $CF_3$, $C_1$-$C_6$ alkyl, $C_5$-$C_{12}$ cycloalkyl, aryl, substituted aryl, heteroaryl or $C_1$-$C_6$ alkoxy, or NR", wherein $NR^{II}$ is a tertiary amine, wherein the tertiary amine is part of a linear or cyclic substructure.

More preferred is a process for preparing a Δ9,11 steroid of the formula Ib,

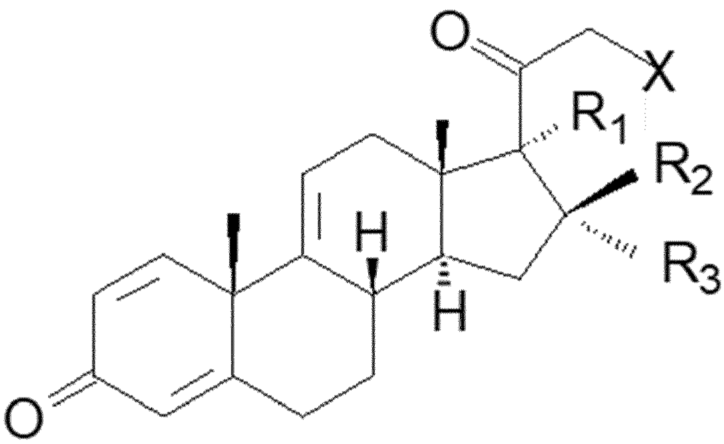

Ib

wherein:

$R^1$ is H or OH; one of $R^2$ or $R^3$ is $CH_3$ and the other is H or both are H; and X is H, halo, OR, wherein R is H or C(O)$R^I$, and $R^I$ is $CF_3$, $C_1$-$C_6$ alkyl, $C_5$-$C_{12}$ cycloalkyl, aryl, substituted aryl, heteroaryl or $C_1$-$C_6$ alkoxy, or $NR^{II}$, wherein $NR^{II}$ is a tertiary amine, wherein the tertiary amine is part of a linear or cyclic substructure.

Even more preferred is a process for preparing a Δ9,11 steroid of the formula Ic,

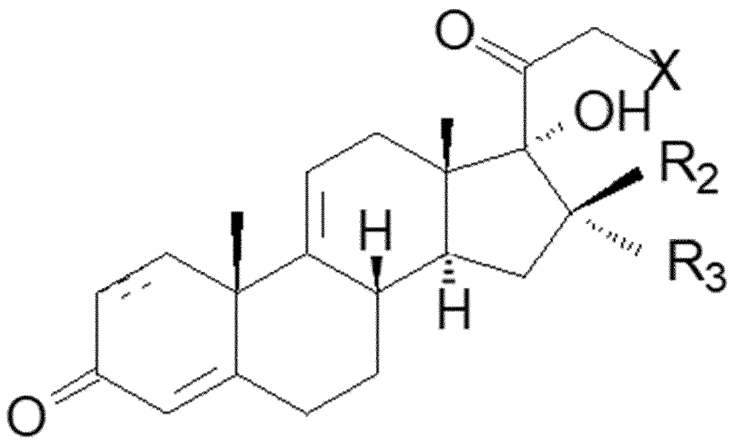

Ic

wherein:

The dotted line is a single or a double bond; one of $R^2$ or $R^3$ is $CH_3$ and the other is H or both are H; and X is H, halo, OR, wherein R is H or C(O)$R^I$, and $R^I$ is $CF_3$, $C_1$-$C_6$ alkyl, $C_5$-$C_{12}$ cycloalkyl, aryl, substituted aryl, heteroaryl or $C_1$-$C_6$ alkoxy, or $NR^{II}$, wherein $NR^{II}$ is a tertiary amine, wherein the tertiary amine is part of a linear or cyclic substructure.

Even more preferred is a process for preparing a ∆9,11 steroid of the formula Id,

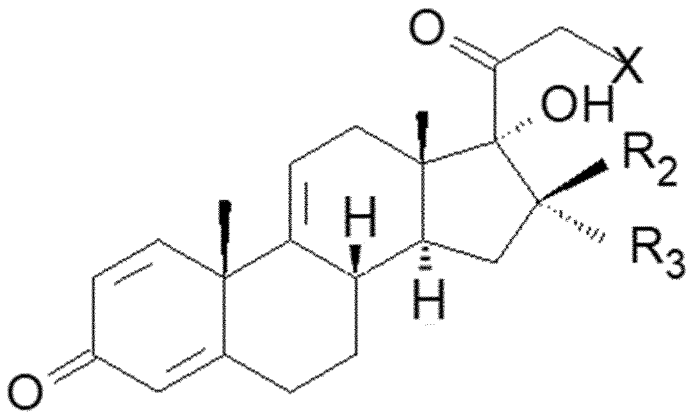

Id

wherein:

One of $R^2$ or $R^3$ is $CH_3$ and the other is H or both are H; and X is H, halo, OR, wherein R is H or C(O)$R^I$, and $R^I$ is $CF_3$, $C_1$-$C_6$ alkyl. $C_5$-$C_{12}$ cycloalkyl, aryl, subst. aryl, heteroaryl or $C_1$-$C_6$ alkoxy, or $NR^{II}$, wherein $NR^{II}$ is a tertiary amine, wherein the tertiary amine is part of a linear or cyclic substructure.

Even more preferred is a process for preparing a ∆9,11 steroid of the formula Ie,

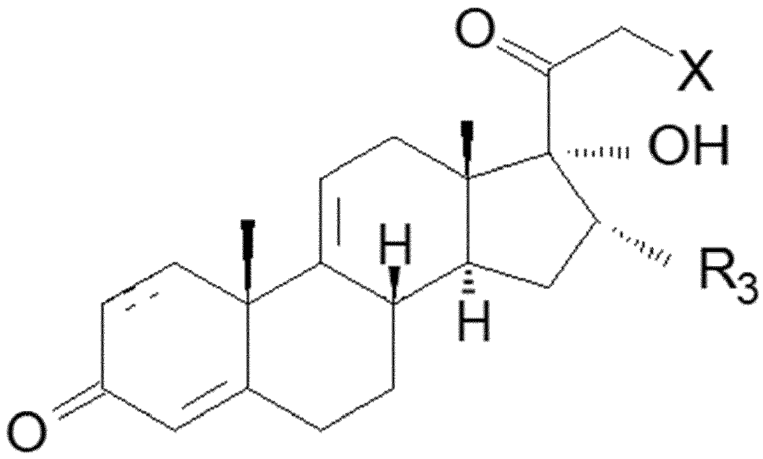

Ie

wherein:

The dotted line is a single or a double bond; $R^3$ is $CH_3$ or H; and X is H, halo, OR, wherein R is H or C(O)$R^I$, and $R^I$ is $CF_3$, $C_1$-$C_6$ alkyl, $C_5$-$C_{12}$ cycloalkyl, aryl, heteroaryl or $C_1$-$C_6$ alkoxy, or $NR^{II}$, wherein $NR^{II}$ is a tertiary amine, wherein the tertiary amine is part of a linear or cyclic substructure.

Even more preferred is a process for preparing a Δ9,11 steroid of the formula If,

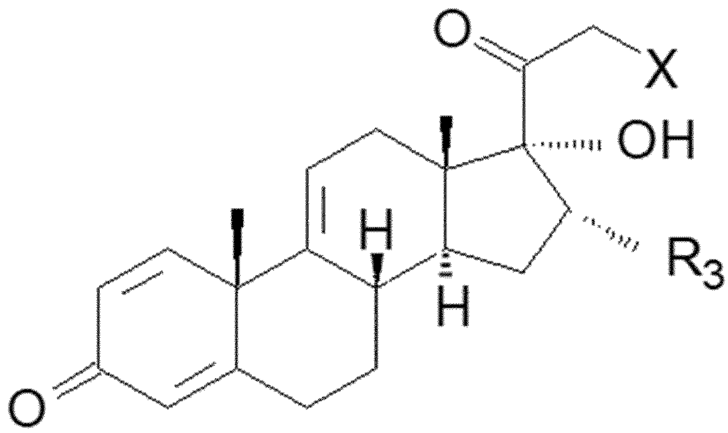

If

wherein:

$R^3$ is $CH_3$ or H; and X is H, halo, OR, wherein R is H or C(O)$R^I$, and $R^I$ is $CF_3$, $C_1$-$C_6$ alkyl, $C_5$-$C_{12}$ cycloalkyl, aryl, heteroaryl or $C_1$-$C_6$ alkoxy, or $NR^{II}$, wherein $NR^{II}$ is a tertiary amine, wherein the tertiary amine is part of a linear or cyclic substructure.

Particularly preferred is a process for preparing a Δ9,11 steroid of any of formulas Ia-If as defined above, wherein X is H, OH or OAc.

Most preferred is a process for preparing one of the Δ9,11 steroids:

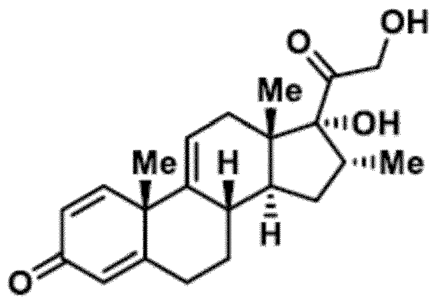

Vamorolone

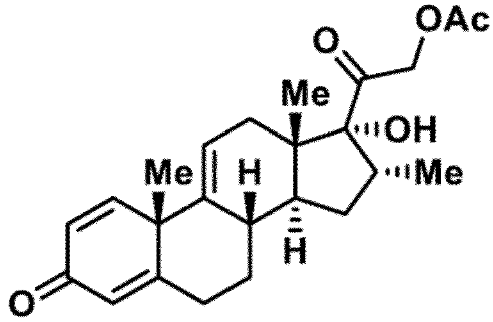

Vamorolone-acetate

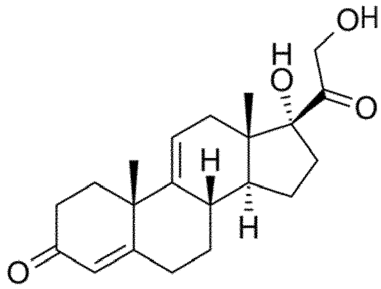

VBP1

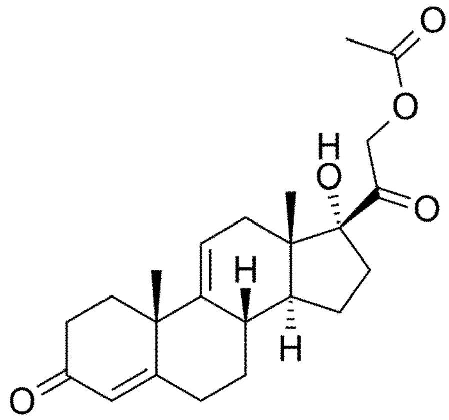

Anecortave

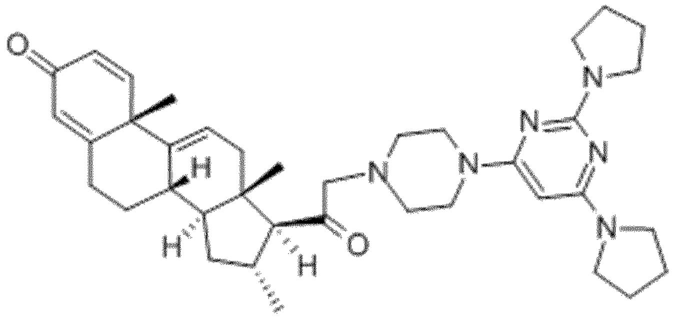

Tirilazad

The instantly claimed process comprises treating an 9,11-epoxy-steroid of the formula II,

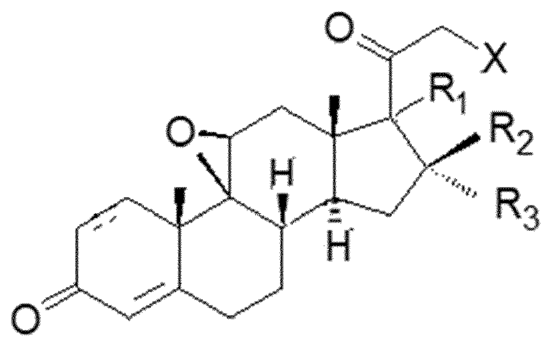

II

wherein the dotted line is a single or a double bond; $R^1$ is H or OH; one of $R^2$ or $R^3$ is $CH_3$ and the other is H or both are H; and X is H, halo, OR, wherein R is H or $C(O)R^I$, and $R^I$ is $CF_3$, $C_1$-$C_6$ alkyl, $C_5$-$C_{12}$ cycloalkyl, aryl, heteroarylor $C_1$-$C_6$ alkoxy, or $NR^{II}$, wherein $NR^{II}$ is a tertiary amine, wherein the tertiary amine is part of a linear or cyclic substructure, with hydroiodic acid, HI, to form the compound of formula I.

Preferably, the instantly claimed process comprises treating an 9,11-epoxy-steroid of the formula IIa

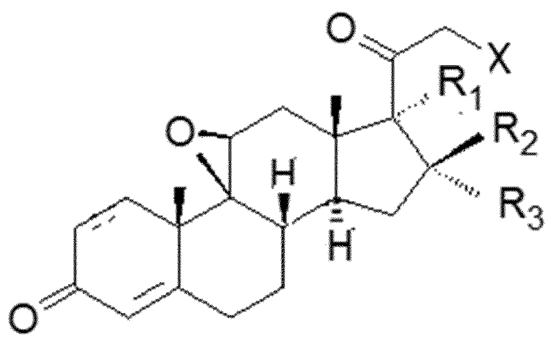

IIa

wherein the dotted line is a single or a double bond; $R^1$ is H or OH; one of $R^2$ or $R^3$ is $CH_3$ and the other is H or both are H; and X is H, halo, OR, wherein R is H or $C(O)R^I$, and $R^I$ is $CF_3$, $C_1$-$C_6$ alkyl, $C_5$-$C_{12}$ cycloalkyl, aryl, heteroarylor $C_1$-$C_6$ alkoxy, or $NR^{II}$, wherein $NR^{II}$ is a tertiary amine, wherein the tertiary amine is part of a linear or cyclic substructure, with hydroiodic acid, HI, to form the compound of formula Ia.

More preferably, the instantly claimed process comprises treating an 9,11-epoxy-steroid of the formula IIb

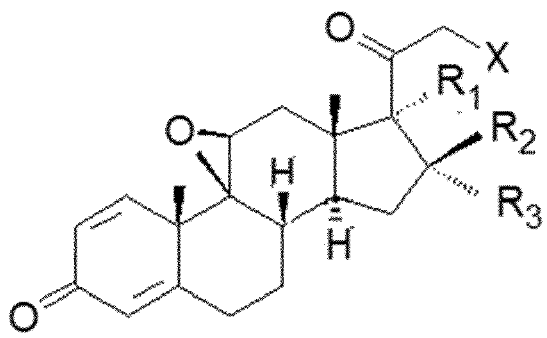

IIb

wherein $R^1$ is H or OH; one of $R^2$ or $R^3$ is $CH_3$ and the other is H or both are H; and X is H, halo, OR, wherein R is H or $C(O)R^I$, and $R^I$ is $CF_3$, $C_1$-$C_6$ alkyl, $C_5$-$C_{12}$ cycloalkyl, aryl, heteroaryl or $C_1$-$C_6$ alkoxy, or NR", wherein $NR^{II}$ is a tertiary amine, wherein the tertiary amine is part of a linear or cyclic substructure, with hydroiodic acid, HI, to form the compound

of formula Ib.

Even more preferably, the instantly claimed process comprises treating an 9,11-epoxy-steroid of the formula IIc

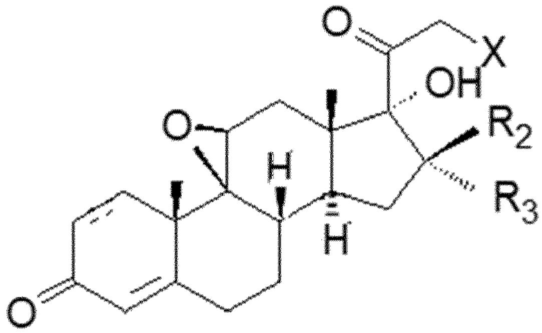

IIc

wherein the dotted line is a single or a double bond; one of $R^2$ or $R^3$ is $CH_3$ and the other is H or both are H; and X is H, halo, OR, wherein R is H or C(O)$R^I$, and $R^I$ is $CF_3$, $C_1$-$C_6$ alkyl, $C_5$-$C_5$-$C_{12}$ cycloalkyl, aryl, heteroarylor $C_1$-$C_6$ alkoxy, or $NR^{II}$, wherein $NR^{II}$ is a tertiary amine, wherein the tertiary amine is part of a linear or cyclic substructure, with hydroiodic acid, HI, to form the compound of formula Ic.

Even more preferably, the instantly claimed process comprises treating an 9,11-epoxy-steroid of the formula IId

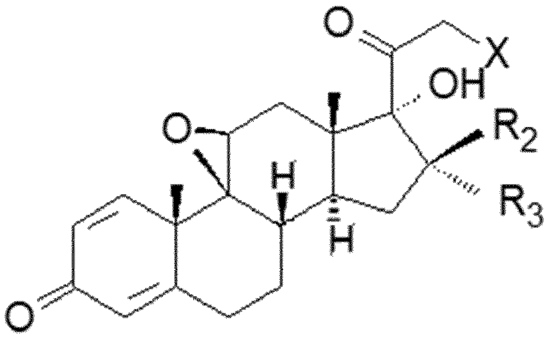

IId

Wherein one of $R^2$ or $R^3$ is $CH_3$ and the other is H or both are H; and X is H, halo, OR, wherein R is H or C(O)$R^I$, and $R^I$ is $CF_3$, $C_1$-$C_6$ alkyl, $C_5$-$C_{12}$ cycloalkyl, aryl, heteroarylor $C_1$-$C_6$ alkoxy, or $NR^{II}$, wherein $NR^{II}$ is a tertiary amine, wherein the tertiary amine is part of a linear or cyclic substructure, with hydroiodic acid, HI, to form the compound of formula Id.

Even more preferably, the instantly claimed process comprises treating an 9,11-epoxy-steroid of the formula IIe

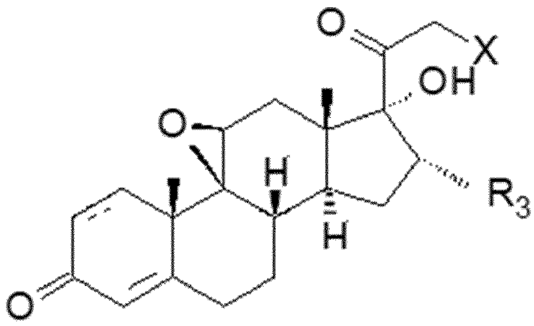

IIe

wherein the dotted line is a single or a double bond; $R^3$ is $CH_3$ or H; and X is H, halo, OR, wherein R is H or C(O)$R^I$,

and $R^I$ is $CF_3$, $C_1$-$C_6$ alkyl, $C_5$-$C_{12}$ cycloalkyl, aryl, heteroarylor $C_1$-$C_6$ alkoxy, or $NR^{II}$, wherein $NR^{II}$ is a tertiary amine, wherein the tertiary amine is part of a linear or cyclic substructure, with hydroiodic acid, HI, to form the compound of formula Ie.

Even more preferably, the instantly claimed process comprises treating an 9,11-epoxy-steroid of the formula IIf,

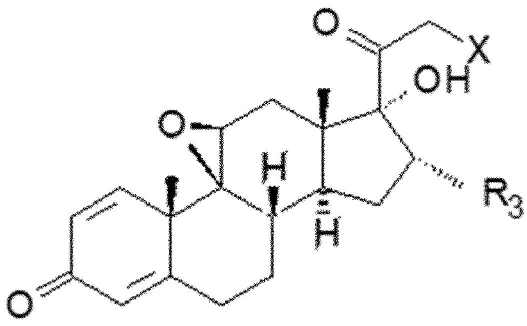

IIf

wherein $R^3$ is $CH_3$ or H; and X is H, halo, OR, wherein R is H or $C(O)R^I$, and $R^I$ is $CF_3$, $C_1$-$C_6$ alkyl, $C_5$-$C_{12}$ cycloalkyl, aryl, heteroaryl or $C_1$-$C_6$ alkoxy, or NR", wherein $NR^{II}$ is a tertiary amine, wherein the tertiary amine is part of a linear or cyclic substructure, with hydroiodic acid, HI, to form the compound of formula If.

More preferably, the instantly claimed process comprises treating a 9,11-epoxy-steroid of VBP1, Anecortave and Tirilazad, respectively, with with hydroiodic acid, HI, to form VBP1, Anecortave and Tirilazad, respectively.

Most preferably, the instantly claimed process comprises treating an 9,11-epoxy-steroid, which is 8-DM or 8-DM-acetate

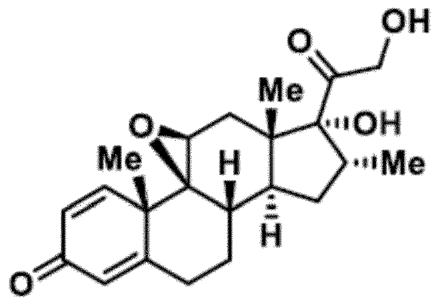

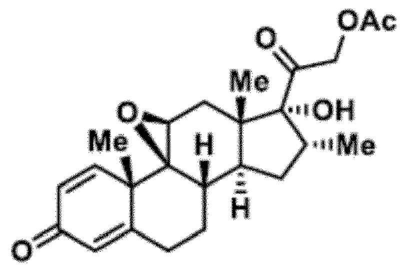

*8-DM* *8-DM-acetate*

with hydroiodic acid, HI, to form Vamorolone and Vamorolone-acetate, respectively.

DETAILS OF THE INVENTION

As used herein, the term "alkyl" means a straight or branched unsaturated hydrocarbon chain; preferred are $C_1$-$C_6$ alkyl having from 1 to 6 carbon atoms. The definition of alkyl and $C_1$-$C_6$-alkyl includes for example the meanings methyl, ethyl, n-, isopropyl, n-, iso-, sec- and t-butyl, n-pentyl, n-hexyl, 1,3-dimethylbutyl, and 3,3-dimethylbutyl.

The term "alkoxy" refers to an alkyl (carbon and hydrogen chain) group singularly bonded to oxygen, R-O; preferred are $C_1$-$C_6$ alkoxy groups having from 1 to 6 carbon atoms.

The term "halo" preferably means bromo, chloro or iodo.

"Cycloalkyl" groups in connection with the present invention are, unless defined otherwise, ring-shaped saturated hydrocarbon groups. Preferred are $C_5$-$C_{12}$ cycloalkyl having 5-12 ring C-atoms

"Aryl" in connection with the present invention is, unless defined otherwise, an aromatic hydrocarbon. Preferred is phenyl or substituted phenyl. "The term "heteroaryl" defines an aryl, which can have one, two or more heteroatoms which are selected from O, N, P and S and can optionally be substituted by further groups. Aryl may be substituted or unsubstituted. "Substituted aryl" means aryl as defined above but containing one or more substuituents. Preferred are substituted aryl containing one or more alkoxy substituents, for instance methoxy groups, and/ or one or more halogens, such as Cl. Heteroaryl may be substituted or unsubstituted. "Substituted heteroaryl" means heteroaryl as defined above

but containing one or more substuituents. Preferred are substituted heteroaryl containing one or more alkoxy substituents, for instance methoxy groups, and/ or one or more halogens, such as Cl.

The term "tertiary amine" denotes an amino group in which the nitrogen atom is bonded to three organic radicals, where two of these radicals may also together be part of a ring. In the present invention, $NR^{II}$ is a tertiary amine, wherein the tertiary amine is part of a linear or cyclic substructure. A preferred tertiary amine is a substituted or unsubstituted piperazin moiety, e.g.

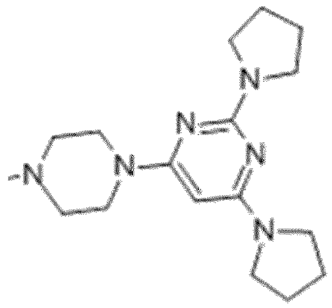

.

The phrase "organic phase" is abbreviated in this specification by "OP", "aqueous phase" by "AP".

The term "pharmaceutical purity" defines an at least 99 wt% pure compound, e.g. Δ9,11 steroid, of the present invention, as determined by HPLC or other conventional methods. More preferably, the compound, e.g. Δ9,11 steroid, of the present invention, is at least 99,5 wt% pure as determined by HPLC or other conventional methods. Most preferably, the compound, e.g. Δ9,11 steroid, of the present invention, is at least 99,9 wt% pure as determined by HPLC or other conventional methods.

The following solvents and reagents employed in the process of the present invention are identified by the abbreviations indicated: ethyl acetate (EtOAc); acetic acid (HOAc); tetrahydrofuran (THF); dimethylsulfoxide (DMSO); triethylamine ($Et_3N$); diisopropylethylamine (Hünigs base); methanol (MeOH); 1,8-diazabicyclo[5.4.0]undec-7-ene (DBU); triphenylphosphine ($PPh_3$); diisopropyl ether ($iPr_2O$); dimethoxyethane (DME); t-butylmethyl ether (t-BuOMe); N,N-dimethylaminopyridine (DMAP); dimethylformamide (DMF); p-toluenesulfonyl chloride (TsCl); $Bu_3Sn_2O$ (TBTO) Polymethylhydrosiloxane (PMHS).

The present invention comprises a process, designated Process A, deoxygenating the steroid of the formula II to form a Δ9,11 steroid of the formula I, as shown in Reaction Scheme A.

Reaction Scheme A

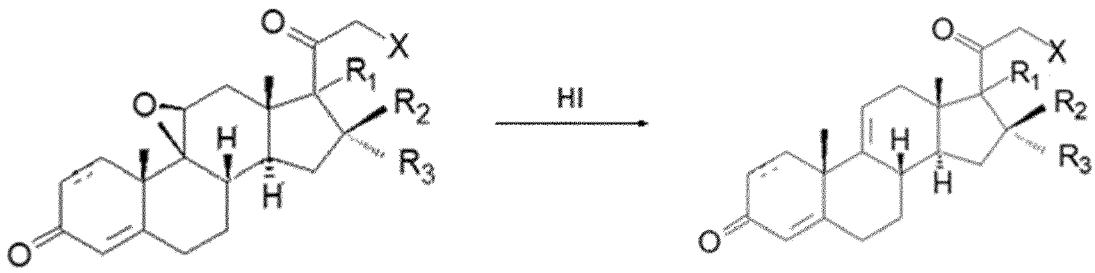


II I

In Reaction Scheme A, a compound of formula II is treated with HI to form the compound of formula I. The present invention further comprises the process of Reaction Scheme A as shown above, wherein a compound of the formula IIa, II, IIc, IId or IIe is used instead of the compound of formula II.

As an example, Reaction Scheme A' is shown. In Reaction Scheme A', a compound of formula IId is treated with HI to form the compound of formula Id.

Reaction Scheme A'

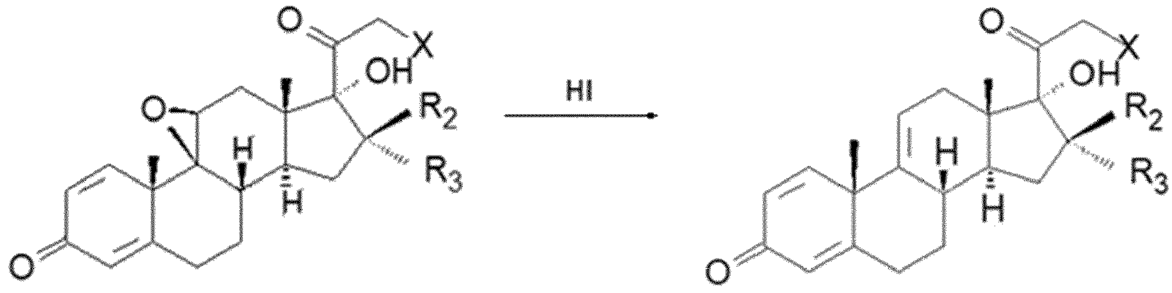


IId Id

Most preferred is the synthesis of Vamorolone and Vamorolone Acetate (see Reaction Schemes B and B'), respectively.

Reaction Scheme B: Synthesis of Vamorolone Acetate

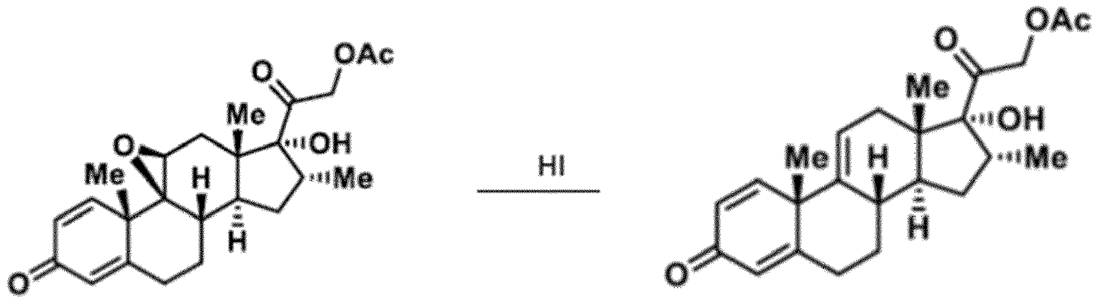


8-DM ACETATE VAMOROLONE ACETATE

In Reaction Scheme B, 8-DM Acetate is treated with HI to form Vamorolone Acetate.

Reaction Scheme B': Synthesis of Vamorolone

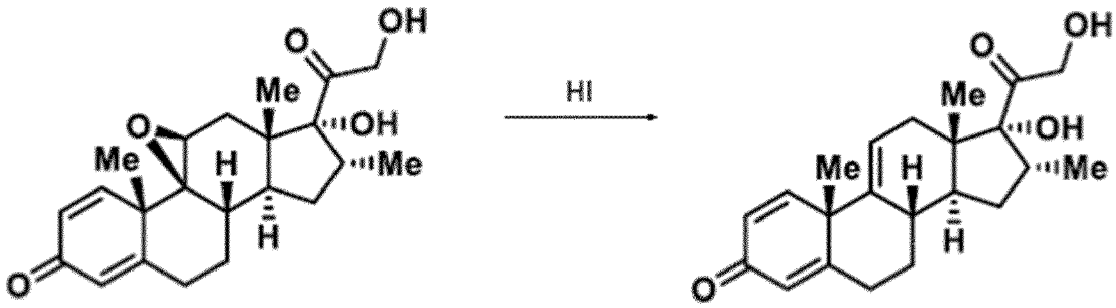


8-DM VAMOROLONE

In Reaction Scheme B', 8-DM is treated with HI to form Vamorolone. Surprisingly, the primary alcohol (21-hydroxy in 8-DM) does not undergo transformation to a corresponding alkyl iodide (21-1).

According to the present invention, the deoxygenation reactions of the present invention with HI can take place

in any organic solvent. The solvent choice, however, is limited in as far as the solvent needs to be stable towards strong acid (HI) and the potentially formed I2, and should also not interfere with any of the reaction intermediates (e.g. iodohydrin). This reduces the number of solvent choices.

Any aromatic solvent, any chlorinated solvent ($CH_2Cl_2$, chloroform, 1,2-DCE etc.) and any nitrile solvent or mixtures of any of these solvents can be used. Preferred aromatic solvents are toluene, xylene, benzene, $PhCF_3$. Preferred chlorinated solvents are $CH_2Cl_2$, chloroform, 1,2-DCE. Preferred nitrile solvents are MeCN, propionitrile, butyronitrile

Three solvents are most preferred according to the present invention, i.e. toluene, MeCN and $CH_2Cl_2$. These solvents can be used alone or in mixtures of any of the three or a mixture of all tree solvents.

Alcoholic solvents, which are stable in the presence of HI, can be used as well. For example, $CF_3CH_2OH$ and HFIP (hexafluoroisopropanol) may be used.

Etheral solvents, which are stable in the presence of HI, can be used as well. For example, DIPE (di-isopropylether) may be used.

The reaction can also be performed directly in an organic acid without any other solvent, e.g. directly in formic acid, acetic acid (i.e. AcOH), TFA etc. Examples of organic acids according to the present invention are acetic acid, TFA, formic acid, and propionic acid.

More preferably, the organic acid is AcOH. Organic acids, preferably AcOH, are good solvents for the reaction, as they facilitate elimination of the hydroxy-group at C11. However, in this embodiment, preferably an organic solvent (e.g. toluene, MeCN and $CH_2Cl_2$) needs to be added prior to quenching of the formed iodine with aqueous $Na_2SO_3$, to avoid by-product formation of insoluble residues.

Another solvent according to the present invention is $H_2O$, wherein the reaction works as well. Preferably, $H_2O$ is used in a mixture with any of the organic acids as mentioned above. However, in $H_2O$ without organic acids, I2 will be formed and will precipitate from $H_2O$. This creates the need of removing precipitated I2, quantitatively.

The reagent hydroiodic acid, HI, is preferably used in a high concentration aqueous form, more preferably in a concentration of 1%-70%, preferably 5%-70%, more preferably 10%-70%, even more preferably 30%-70% aqueous HI by mass, most preferably 48-57% aqueous HI or 64%-68% aqueous HI by mass.

The deoxygenation reaction with hydroiodic acid is performed at temperatures below room temperature RT (i.e. 25°C), preferably below 15°C, more preferably below 10 °C, most preferably below 5°, e.g. between 1 and 5°C.

To purify the compound of formula II, the final compound is recrystallized. Recrystallization may be performed from polar solvents such as water or alcohols and mixtures thereof and alternatively using Acetonitrile, Acetone, Methyl ethyl ketone (MEK), Methyl isopropyl ketone (MIK) and mixtures thereof. Preferably, re-crystallization is performed from isopropanol (iPr-OH) or a mixture of iPr-OH and water. More preferably recrystallization takes place in a mixture of iPr-OH : water between 60:40 wt% and 100 : 0 wt%, preferably between 80 : 20 wt% and 100 : 0 wt%

Typically the last crystallization of crude Vamorolone from isopropanol or isopropanol water mixtures is able to provide a purity upgrade of more than 1 wt%, e.g. from 98.5 wt% to between 99.6 wt% and 99.9 wt% while still providing good yields above 90%. This final polishing step by crystallization allows excellent control of the impurity profile of the final API.

In a further aspect of the present invention a protecting group for the primary hydroxy group on C-21 can be added in place of moiety X before the deoxygenation of formula II, IIa, IIb, IIc, IId, IIe or IIf, respectively.

The protecting group in place of X, is OR, wherein R is $C(O)R^I$, wherein $R^I$ is $CF_3$, $C_1$-$C_6$ alkyl, $C_5$-$C_{12}$ cycloalkyl, aryl, heteroaryl or $C_1$-$C_6$ alkoxy.

After the addition of the protecting group, the deoxygenation according to the present invention is performed, followed by a de-protection step, i.e. a step in which the protecting group is removed.

In this step moiety X, which is OR, wherein R is $C(O)R^I$, and $R^I$ is $CF_3$, $C_1$-$C_6$ alkyl, $C_5$-$C_{12}$ cycloalkyl, aryl, heteroaryl or $C_1$-$C_6$ alkoxy, is converted into X, which is OH. OH can furthermore be protected using methoxymethyl ether, tetrahydropyranyl ether, *t*-butyl ether, benzyl ether, dimethoxybenzyl ether, *t*-butyldimethylsilyl ether, *t*-butyldiphenylsilyl ether, acetic acid ester, pivalic acid ester, benzoic acid ester, acetonide or benzylidene acetal or any other conventional protecting groups.

In a preferred embodiment, 8-DM is acetylated before the deoxygenation with HI. The obtained 8-DM Acetate is deoxygenated with HI to Vamorolone Acetate and subsequently de-acetylated to form Vamorolone. If Vamorolone Acetate is partially deacetylated during the HI reaction, it is optional to re-acetylate the Vamorolone partially de-acetylated, again. Quantitative acetylation may be intended to quantitatively isolate Vamorolone Acetate and obtain a quantitative total synthesis of Vamorolone, if the synthesis involves Vamorolone Acetate formation by adding HI to 8-DM acetate.

The acetylation procedure can be performed using known reaction conditions specified in this patent in the experimental section or conditions known to the skilled person. However, particularly preferred is a reaction using $Ac_2O$ in an organic solvent, preferably in Acetonitrile. Most preferably, $Ac_2O$ in Acetonitrile is used with catalytic amounts of DMAP. The 8-DM-Acetate may be obtained as a crystalline product after aqueous quenching. The acetylation may be performed at room temperature. Preferred temperature ranges are between 20 and 30°C, more preferably 22-25°C

The de-acetylation hydrolysis procedure can be performed using reactions known in the art. However, particularly preferred is de-acetylation with $K_2CO_3$ in an alcohol or an alcohol / water-mixture. Most preferred is $K_2CO_3$ in $MeOH/H_2O$. In a further embodiment, Vamorolone Acetate is de-acetylated with KOH in an alcohol or an alcohol / water-mixture. Most preferred is KOH in MeOH.

The temperature of the de-acetylation reaction is lower than RT, preferably lower than 15°C, more preferably lower than 10°C, most preferably between 0°-5°C.

In a preferred embodiment of the present invention, the Δ9,11 steroid is prepared by deoxygenation of a respective 9,11-epoxy steroid using HI, wherein the Δ9,11 steroid is prepared using aqueous HI at a concentration of between 48 and 68 wt%, in MeCN, $CH_2Cl_2$, or Toluene; or a mixture of two or all three thereof, in the presence or absence of an organic acid, wherein treatment with HI is carried out at a temperature of below 15°C.

In a more preferred embodiment of the present invention, the Δ9,11 steroid is prepared by deoxygenation of a respective 9,11-epoxy steroid using HI, wherein the Δ9,11 steroid is prepared using aqueous HI at a concentration of between 48 and 68 wt%, in MeCN, $CH_2Cl_2$, or Toluene; or a mixture of two or all three thereof, in the presence or absence of an organic acid, which is acetic acid, wherein treatment with HI is carried out at a temperature of below 15°C.

In an even more preferred embodiment of the present invention, the Δ9,11 steroid is prepared by deoxygenation of a respective 9,11-epoxy steroid using HI, wherein the Δ9,11 steroid is prepared using aqueous HI at a concentration of between 48 and 68 wt%, inToluene; in the presence or absence of an organic acid, which is acetic acid, wherein treatment with HI is carried out at a temperature of below 15°C.

In an even more preferred embodiment of the present invention, the Δ9,11 steroid is prepared by deoxygenation of a respective 9,11-epoxy steroid using HI, wherein the Δ9,11 steroid is prepared using aqueous HI at a concentration of between 48 and 68 wt%, in toluene; in the presence or absence of an organic acid, which is acetic acid, wherein treatment with HI is carried out at a temperature of below 15°C, wherein if X is OH, the 9,11 epoxy steroid is acetylated before the treating of the 9,11 epoxy steroid with HI. In this embodiment, the Δ9,11 steroid obtained after treating the 9,11 epoxy steroid with HI, is preferably de-acetylated.

Most preferably, the processes of the present invention produce Vamorolone or Vamorolone Acetate, more preferably from the commercial 8-DM.

The Δ9,11 steroids obtained by the processes of the present invention are ideally pharmaceutically pure Δ9,11 steroids. Purification can be performed by recrystallization of any of the Δ9,11 steroids obtained by any of the processes of the present invention, which are typically obtained as crude Δ9,11 steroids, in iPrOH or a mixture of iPrOH and water.

## EXAMPLES

### Example 1: Unsuccessful Attempts

#### 1.1 Unsuccessful conditions

Deoxygenation was tried but found to be unsuccessful with the following reactions:

a) Rhenium-catalysts: $CH_3ReO_3$, $Re_2O_7$ or perrhenic acid) in combination with Triphenylphosphite $P(OPh)_3$ in toluene at reflux,

b) Electrochemical Reduction with aq. $NH_4Br$ / THF with Zn-electrodes. Constant Current, 30mA, and

c) $PPh_3$ & I2 in MeCN.

None of these reactions known from the literature turned out to be successful.

#### 1.2 Useful but complex and toxic reaction with HBr

Alternative deoxygenation conditions using HBr followed by tin mediated PMHS reduction and subsequent water elimination involves large quantities of a tin reagent and liquid sulfur dioxide as the solvent for the elimination step. Neither the use of tin nor toxic gases as solvents or reagents is desirable for the downstream manufacturing of an API if it can be avoided.

Thus there is a need for a new process to circumvent the use of hazardous and toxic chemicals towards the end of the chemical sequence to Vamorolone.

Furthermore, the HBr route to Vamorolone has the potential to form PMIs (Potentially Mutagenic Impurities) such as allylic bromides of Vamorolone that could represent a patient risk. These impurities are hard to trace and must be controlled rigorously to very low contamination levels.

Scheme II:

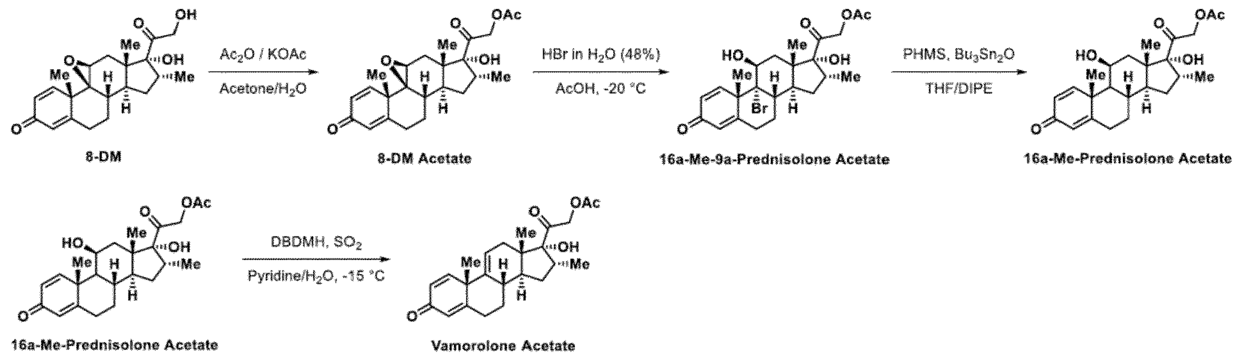


The steps of scheme 2 turned out to be possible, but unintended for several reasons: Debromination with $Bu_2Sn_2O$ comprises the *in situ* generation of $Bu_3SnH$, which is toxic. $Bu_3Sn_2O$ (TBTO) used as biocid in ship paintings to reduce biofouling. TBT is highly toxic itself towards nontarget organisms. Toxic effects occur already at 1 nano-gram per liter of water. $SO_2$ is toxic and it represents a safety hazard in a manufacturing plant and needs specific safety installations, controls and waste treatment facilities.

### 1.3 Useful but imperfect reaction with "TMSI"

Deoxygenation was tried, but found to be uncomfortable, by use of NaI (3.0 eq.) & TMSCl (1.5 eq.) in MeCN. After in situ formation of TMS-iodohydrin, and subsequent elimination the deoxygenation took place:
However, incomplete transformation of 8-DM-Acetate and a non-satisfactory resulting impurity profile required a new solution.Thus, the inventors sought to provide a new, even simpler and more straight forward and scalable synthesis.

## Example 2: Synthesis of the present invention

Scheme C: Route of Synthesis of Vamorolone from 8-DM

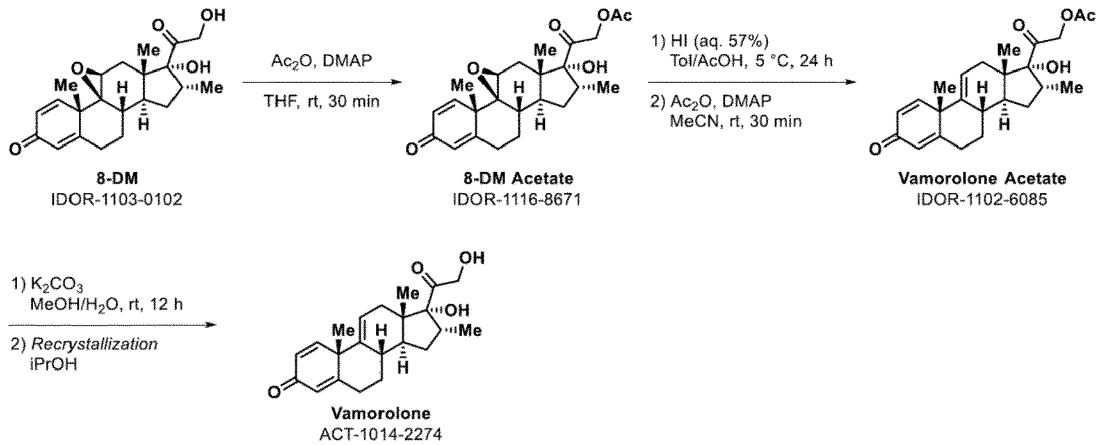


Vamorolone was synthesized in three synthetic steps from commercially available 8-DM.

The synthetic route started with the acetylation of 8-DM using acetic anhydride and catalytic DMAP in THF, followed by crystallization of 8-DM Acetate after aqueous quench. Then, a deoxygenation reaction converted 8-DM Acetate directly into Vamorolone Acetate. This deoxygenation proceeded via initial formation of an iodohydrin with excess aq. HI, followed by simultaneous I2 and $H_2O$-elimination to give Vamorolone Acetate. During the reaction, partial de-acetylation occurred (20-25%) and therefore re-acetylation with acetic anhydride was necessary. After completed re-acetylation, Vamorolone Acetate was directly crystallized by addition of $H_2O$. Finally, the acetate group is cleaved under

basic conditions to give crude Vamorolone, which was recrystallized from iPrOH to obtain the pure product.

### 2.1 Acetylation

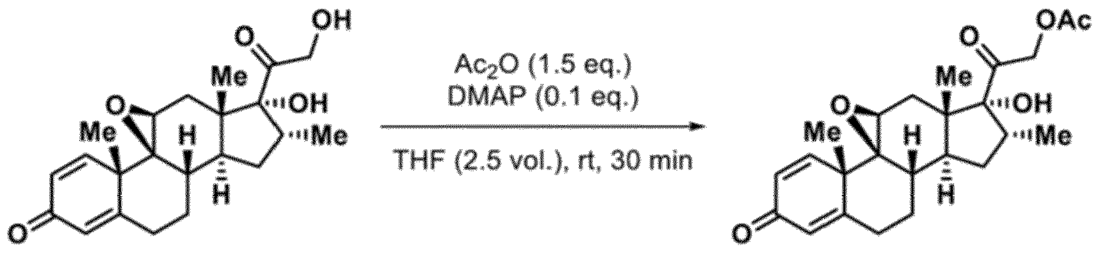


**8-DM** **8-DM Acetate**

A 10 L glass dj (double jacketed reactor)-reactor was charged with **8-DM** (490 g, 1.32 mol, 1.0 eq.) and DMAP (16.1 g, 0.132 mmol, 0.10 eq.). THF (1.25 L, 2.5 vol.) was added at IT = 20-25 °C. Then, $Ac_2O$ (201 g, 187 mL, 1.97 mol, 1.5 eq.) was added dropwise over 20-40 min, keeping IT below 30 °C during the addition. After complete addition, the reaction mixture was stirred at IT = 20-25 °C for 30 min. IPC control by LC/MS indicated >99% conversion of **8-DM to 8-DM Acetate.**

The reaction mixture was quenched by dropwise addition of $H_2O$ (4.9 L, 10 vol.) over 30-45 min, keeping IT below 25 °C. The resulting aqueous suspension was aged at IT = 20-25 °C for 1 h. The product was filtered off, washed with $H_2O$ (3 x 0.5 L), and dried on a rotary evaporator (900-10 mbar, 65 °C bath temperature) to provide **8-DM Acetate** (539 g, 1.30 mol, 99% yield, >99% a/a, 98% w/w) as a white solid (cryst 1#1).

Analytical Data:

LC/MS column: Zorbax RRHD SB-Aq, 2.1x50mm, 1.8μm
Program: G_005%B_TFA_0,800ml_2,00min
Eluent A: Water/TFA 100:0.04, Eluent B: Acetonitrile
IPC preparation for LC/MS
10 microliter in 1 mL H2O:MeCN 1:1
Conversion was determined with respect to consumption of **8-DM** relative to formation of **8-DM Acetate.**
Detected mass: [M+1]= 373.19 for 8-DM and [M+1] = 415,19 8-DM Acetate.

### 2.2 Deoxygenation with HI

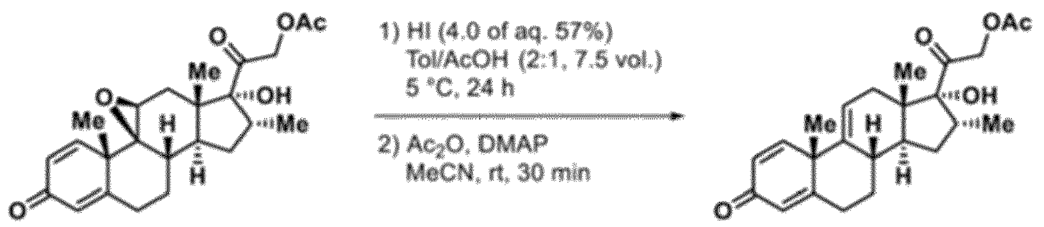


**8-DM Acetate** **Vamorolone Acetate**

A 10 L glass dj-reactor was charged with **8-DM Acetate** (500 g, 1.21 mol, 1.0 eq.). Toluene (2.5 L, 5 vol.) was added. The suspension was cooled to IT = 0-5 °C and then a solution of 57% aqueous HI (1.08 kg, 637 mL, 4.83 mol, 4.0 eq.) in AcOH (1.25 L, 2.5 vol.) was added via peristaltic pump over 45-60 min, keeping IT below 5 °C during the addition. The resulting dark purple to brown solution was stirred at IT = 3-5 °C for 24 h. IPC control by LC/MS indicated

>98% conversion of **8-DM Acetate**/intermediate iodohydrin to **Vamorolone Acetate/Vamorolone.**

The reaction mixture was quenched by dropwise addition of 25% aq. $Na_2SO_3$ solution (2.0 L, 4 vol.) over 10-20 min, keeping IT below 15 °C. After complete addition, EtOAc (1.0 L, 2 vol.) was added and the biphasic mixture was warmed to IT = 15-20 °C. Stirring was stopped and the phases were separated (Organic Phase 1 and aqueous Phase 1; **goal** pH of the aqueous Phase 1: 2; aqueous Phase 1 disposed). 25% aq. $Na_2SO_3$ solution (1.25 L, 2.5 vol.) was added to Organic Phase 1 and the biphasic mixture was stirred at IT = 15-20 °C for 5 min, stirring was stopped and phases separated (Organic Phase 1 and aqueous Phase 2; **goal** pH aqueous Phase 2: 4-5; aqueous Phase 2 disposed). 25% aq. $Na_2SO_3$ solution (1.25 L, 2.5 vol.) was added to Organic Phase 1 and the biphasic mixture was stirred at IT = 15-20 °C for 5 min, stirring was stopped and phases separated (Organic Phase 1 and aqueous Phase 3; **goal** pH aqueous Phase 3: 5-6; aqueous Phase 3 disposed). $H_2O$ (0.5 L, 1.0 vol.) was added to Organic Phase 1 and the biphasic mixture was stirred at IT = 15-20 °C for 5 min, stirring was stopped and phases separated (Organic Phase 1 and aqueous Phase 4; **goal** pH aqueous Phase 4: 5-6; aqueous Phase 4 disposed).

A slight vacuum was applied to the double-jacketed reactor (100-150 mbar), containing Organic Phase 1, and toluene was distilled off at 70 °C jacket temperature (ET) from the reaction mixture with continuous addition of MeCN, and the distillation continued until target residual toluene value has been reached (**goal**: less than **5% toluene** according to 1H-NMR of reaction mixture. Final volume in reactor after distillation: ca. 3.5 L (7.5 vol.).

Once toluene was removed, the vacuum was broken with $N_2$ and resulting fine suspension cooled to IT = 20-25 °C. At this point, the amount of **Vamorolone** was assessed by IPC (typical ratio: **Vamorolone Acetate** to **Vamorolone:** 75:25; x = 25% a/a). DMAP (3.7 g, 0.0302 mol, 0.025 eq.) was added, followed by slow addition of $Ac_2O$ (61.6 g, 57 mL, 0.603 mol, 0.5 eq.) over 5-10 min at IT = 20-25 °C. After complete addition of $Ac_2O$, the reaction mixture was stirred for 30 min at IT = 20-25 °C. IPC control by LC/MS indicated ≤ 2% a/a **Vamorolone** (ratio: **Vamorolone Acetate** to **Vamorolone:** 98.5:1.5).

The reaction mixture was quenched by slow addition of $H_2O$ (4.9 L, 10 vol.) over 15-30 min, keeping IT below 25 °C. The resulting aqueous suspension was cooled to IT = 0-5 °C and aged at this temperature for 2 h. The product was filtered off, washed with $H_2O$/MeCN 4:1 (2 x 0.5 L), and dried on a rotary evaporator (900-10 mbar, 65 °C bath temperature) to provide **Vamorolone Acetate** (301 g, 0.76 mol, 63% yield, 98% a/a, 98% w/w) as an off-white solid (cryst 1#1).

Over the course of the reaction, partial de-acetylation of **Vamorolone Acetate** to **Vamorolone** was observed (between 20-25% a/a). Therefore, after aq. workup and solvent switch to MeCN, the ratio of **Vamorolone Acetate** to **Vamorolone** was assessed by LC/MS (in % a/a), and the following amounts of DMAP and Ac2O were added:

**x = amount of Vamorolone in % a/a (e.g. x = 20% a/a)**

**DMAP eq.** = (0.1·x)/100 (e.g. 0.02 eq.)

**Ac2O eq.** = (2.0·x)/100 (e.g. 0.40 eq.)

Analytical Data

LC/MS column: Zorbax RRHD SB-Aq, 2.1x50mm, 1.8µm
Program: G_005%B_TFA_0,800ml_2,00min
Eluent A: Water/TFA 100:0.04, Eluent B: Acetonitrile
IPC preparation for LC/MS
10 microliter in 1 mL $H_2O$:MeCN 1:1
Conversion was determined with respect to consumption of the sum of **(8-DM Acetate** + intermediate **iodohydrin)** relative to the sum of **(Vamorolone Acetate** + **Vamorolone).**
Detected mass: [M+1] = 415,19 for 8-DM Acetate, [M+1]= 357,28 for Vamorolone, 399,20 for Vamorolone Acetate and 543,12 for intermediate Iodohydrin

**2.3 De-Acetylation**

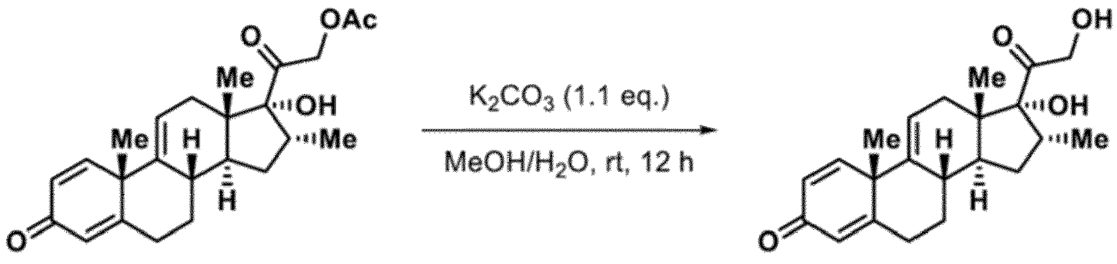


**Vamorolone Acetate** **Vamorolone**

A 10 L glass dj-reactor was charged with **Vamorolone Acetate** (280 g, 0.703 mol, 1.0 eq.). MeOH (1.54 L, 5.5 vol.) was added. The suspension was cooled to IT = 0-5 °C and then a solution of $K_2CO_3$ (107 g, 0.773 mol, 1.1 eq.) in $H_2O$ (0.7 L, 2.5 vol.) was added dropwise via peristaltic pump over 20-40 min, keeping IT below 10 °C during the addition. After complete addition, the reaction mixture was warmed IT = 20-25 °C and stirred for 5 h. IPC control by LC/MS indicated 99.3% conversion of **Vamorolone Acetate** to **Vamorolone.**

The reaction mixture was cooled to IT = 15-17 °C and quenched by dropwise addition of 1 M aq. HCl (950 mL, 0.95 mol, 1.35 eq.) over 20-40 min, keeping IT below 20 °C during the addition **(goal** pH: 5-6). The resulting aqueous suspension was aged at IT = 15-20 °C for 12 h. The product was filtered off, washed with $H_2O$/MeOH 2:1 (3 x 0.3 L), and dried on a rotary evaporator (900-10 mbar, 65 °C bath temperature) to provide **Vamorolone** (241.5 g, 0.68 mol, 96% yield, >99% a/a, 98% w/w) as a slightly yellow solid (crude 1#1).

Analytical Data

LC/MS column: Zorbax RRHD SB-Aq, 2.1x50mm, 1.8μm
Program: G_005%B_TFA_0,800ml_2,00min
Eluent A: Water/TFA 100:0.04, Eluent B: Acetonitrile
IPC preparation for LC/MS
10 microliter in 1 mL $H_2O$:MeCN 1:1
Conversion was determined with respect to consumption of **Vamorolone Acetate** relative to formation of **Vamorolone.**

**2.4 Recrystallization**

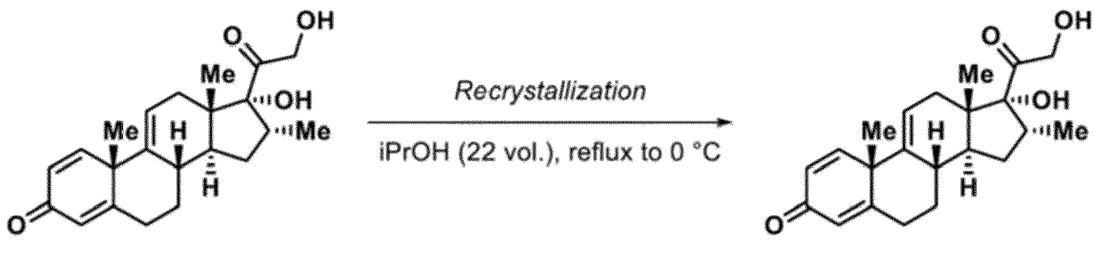


**Vamorolone** **Vamorolone**

A 10 L glass dj-reactor was charged with **Vamorolone** (230 g, 0.645 mol, 1.0 eq.). iPrOH (5 L, 22 vol.) was added. The suspension was heated to reflux (jacket temperature ET = 97 °C) and stirred until complete dissolution of **Vamorolone** occurred (10-15 min on this scale). After complete dissolution, the clear yellow solution was slowly cooled to IT = 0-5 °C over the course of 12 h and then aged at IT = 0-5 °C for 1 h. The recrystallized product was filtered off, washed with cold iPrOH (2 x 250 mL), and dried on a rotary evaporator (900-10 mbar, 65 °C bath temperature) to provide Vamorolone (201 g, 87% recovery, >99% a/a, 99% w/w) as an off white glimmery solid (cryst 1#1).

Iso-propanol (iPrOH) was found to the best solvent for recrystallization with excellent purity upgrading properties (by rejection of impurities), although a high dilution is necessary to completely dissolve the crude Vamorolone at reflux temperature. Higher concentrations for the recrystallization satisfactory results are obtainable using mixtures of isopropanol and water. Maximum solubility of Vamorolone was determined to be at reflux of a 80:20 (isopropanol: water) mixture.

**2.5 Improving purity by Charcoal treatment**

To improve Vamorolone Acetate purity, a charcoal treatment was envisaged. Two options were found to be useful.

**Option 1:** Charcoal treatment on isolated Vamorolone Acetate

Vamorolone Acetate (10 g) from HI step (ELN293-1469.1) was suspended in MeCN (100 mL, 10 vol.) and H2O (10 mL, 1 vol.). The suspension was heated to IT = 60-65 °C and stirred until Vamorolone Acetate was completely dissolved. Then, charcoal (1.0 g, 10% w/w) was added and stirred for 1 h at IT = 60-65 °C. The mixture was filtered hot through a Whatman glass microfiber filter into a 500 mL round-bottom flask. Additional $H_2O$ (90 mL, 9 vol.) was slowly added to induce crystallization of Vamorolone Acetate. The suspension was slowly cooled to 0 °C and aged for 1 h. The white solid was filtered off, washed with additional MeCN:H2O 1:4 (2 x 10 mL) and dried under reduced pressure on the rotavap (65 °C) for extended amount of time (9.0 g, 90% recovery).

**Option 2:** Charcoal treatment during HI step

A 1 L glass dj-reactor was charged with **8-DM Acetate** (30.0 g, 72.4 mmol, 1.0 eq.). Toluene (150 mL, 5 vol.) was added. The suspension was cooled to IT = 0-5 °C and then a solution of 57% aqueous HI (65.0 g, 38.2 mL, 290 mmol, 4.0 eq.) in AcOH (75 mL, 2.5 vol.) was added via peristaltic pump over 45-60 min, keeping IT below 5 °C during the addition. The resulting dark purple to brown solution was stirred at IT = 3-5 °C for 24 h. IPC control by LC/MS indicated >98% conversion of **8-DM Acetate**/intermediate iodohydrin to **Vamorolone Acetate/Vamorolone.**

The reaction mixture was quenched by dropwise addition of 25% aq. $Na_2SO_3$ solution (120 mL, 4 vol.) over 10-20 min, keeping IT below 15 °C. After complete addition, EtOAc (60 mL, 2 vol.) was added and the biphasic mixture was warmed to IT = 15-20 °C. Stirring was stopped and the phases were separated (Organic Phase 1, i.e. OP1 and aqueous Phase 1, i.e. AP1; **goal** pH AP1: 2; AP1 disposed). 25% aq. $Na_2SO_3$ solution (75 mL, 2.5 vol.) was added to OP1 and the biphasic mixture was stirred at IT = 15-20 °C for 5 min, stirring was stopped and phases separated (OP1 and AP2; **goal** pH AP2: 4-5; AP2 disposed). 25% aq. Na2SO3 solution (75 mL, 2.5 vol.) was added to OP1 and the biphasic mixture was stirred at IT = 15-20 °C for 5 min, stirring was stopped and phases separated (OP1 and AP3; **goal** pH AP3: 5-6; AP3 disposed). H2O (30 mL, 1.0 vol.) was added to OP1 and the biphasic mixture was stirred at IT = 15-20 °C for 5 min, stirring was stopped and phases separated (OP1 and AP4; **goal** pH AP4: 5-6; AP4 disposed).

A slight vacuum was applied to the double-jacketed reactor (100-150 mbar), containing OP1, and toluene was distilled off at 70 °C jacket temperature (ET) from the reaction mixture with continuous addition of MeCN, and the distillation continued until target residual toluene value has been reached **(goal:** less than **5% toluene** according to 1H-NMR of reaction mixture. Final volume in reactor after distillation: ca. 225 mL (7.5 vol.))

Once toluene was removed, the vacuum was broken with N2 and resulting fine suspension cooled to IT = 20-25 °C. At this point, the amount of **Vamorolone** was assessed by IPC (ratio: **Vamorolone Acetate** to **Vamorolone:** 75:25; x = 25% a/a). DMAP (221 mg, 1.81 mmol, 0.025 eq.) was added, followed by slow addition of $Ac_2O$ (3.7 g, 3.4 mL, 36.2 mmol, 0.5 eq.) over 2-3 min at IT = 20-25 °C. After complete addition of $Ac_2O$, the reaction mixture was stirred for 30 min at IT = 20-25 °C. IPC control by LC/MS indicated less than 1% a/a **Vamorolone.**

The reaction mixture was quenched by slow addition of $H_2O$ **(30 mL, 1 vol.)** over 5-10 min. The suspension was heated to IT = 60-65 °C and stirred until complete dissolution has occurred. Then, charcoal (3.0 g, 10% w/w) was added and stirred for 1 h at IT = 60-65 °C. The mixture was filtered hot through a Whatman glass microfiber filter into a 1 L round-bottom flask. Additional $H_2O$ (195 mL, 6.5 vol.) was slowly added to induce crystallization of Vamorolone Acetate. The suspension was slowly cooled to 0 °C and aged for 1 h. The white solid was filtered off, washed with additional MeCN:$H_2O$ 1:4 (2 x 40 mL) and dried under reduced pressure at the rotavap (65 °C) for extended amount of time (18.7 g, 46.9 mmol, 65% yield, >99% a/a).

**Claims**

1. Use of hydroiodic acid for the preparation of a $\Delta$9,11 steroid from a 9,11 epoxy steroid.

2. Use of claim 1, wherein the $\Delta$9,11 steroid is a steroid of formula I,

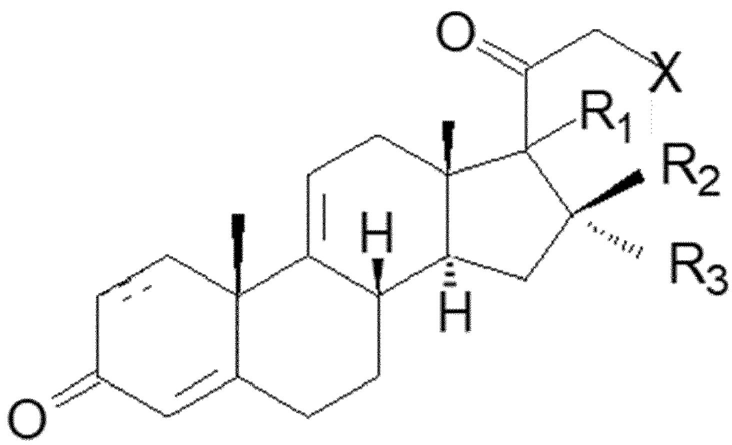

I

wherein the dotted line is a single or a double bond; $R^1$ is H or OH; one of $R^2$ or $R^3$ is $CH_3$ and the other is H or both are H; and X is H, halo, OR, wherein R is H or C(O)$R^I$, and $R^I$ is $CF_3$, $C_1$-$C_6$ alkyl or $C_1$-$C_6$ alkoxy, $C_5$-$C_{12}$ cycloalkyl, aryl, heteroaryl or $NR^{II}$, wherein $NR^{II}$ is a tertiary amine, wherein the tertiary amine is part of a linear or cyclic substructure.

**3.** Use of claim 1 or 2, wherein the Δ9,11 steroid is a steroid of formula If,

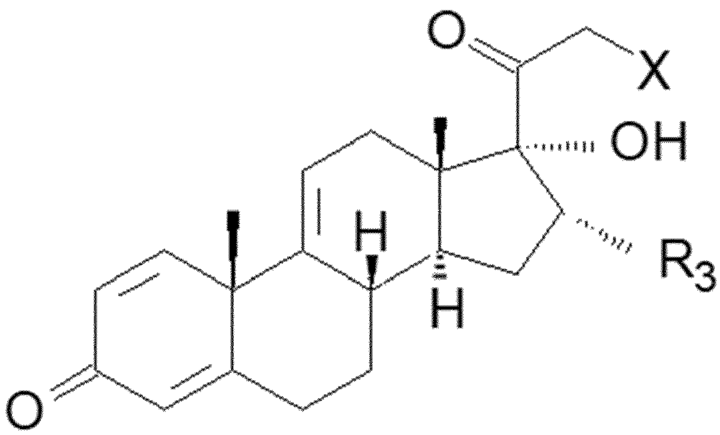

If

wherein $R^3$ is $CH_3$ or H; and X is H, halo, OR, wherein R is H or C(O)$R^I$, and $R^I$ is $CF_3$, $C_1$-$C_6$ alkyl, $C_5$-$C_{12}$ cycloalkyl, aryl, heteroarylor $C_1$-$C_6$ alkoxy, or $NR^{II}$, wherein $NR^{II}$ is a tertiary amine, wherein the tertiary amine is part of a linear or cyclic substructure.

**4.** Use of any of claims 1-3, wherein the Δ9,11 steroid is Vamorolone or Vamorolone Acetate,

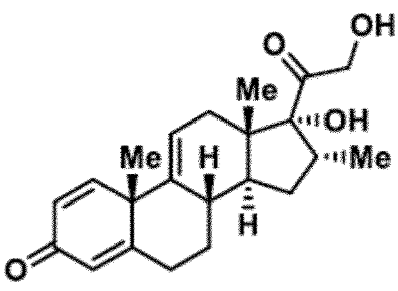

*Vamorolone*

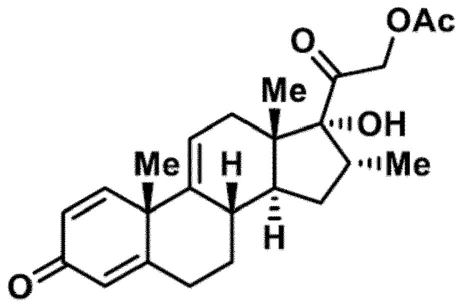

*Vamorolone-acetate.*

**5.** A process for preparing a $\Delta$9,11 steroids of the formula I,

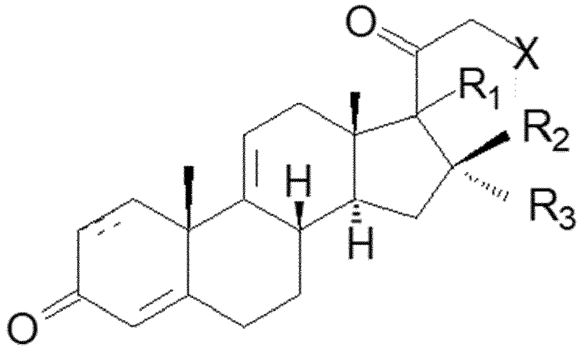

I

wherein the dotted line is a single or a double bond; $R^1$ is H or OH; one of $R^2$ or $R^3$ is $CH_3$ and the other is H or both are H; and X is H, halo, OR, wherein R is H or C(O)$R^I$, and $R^I$ is $CF_3$, $C_1$-$C_6$ alkyl, $C_5$-$C_{12}$ cycloalkyl, aryl, heteroaryl or $C_1$-$C_6$ alkoxy, or $NR^{II}$, wherein $NR^{II}$ is a tertiary amine, wherein the tertiary amine is part of a linear or cyclic substructure,
comprising treating an 9,11 epoxy steroid of the formula II

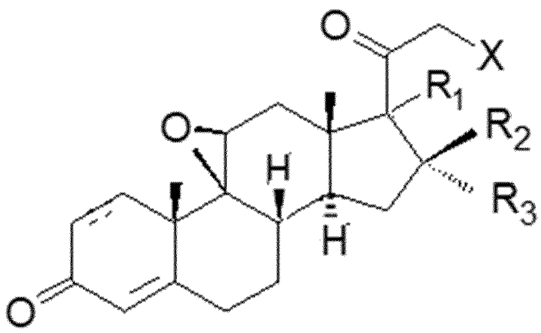

II

wherein the dotted line is a single or a double bond; $R^1$ is H or OH; one of $R^2$ or $R^3$ is $CH_3$ and the other is H or both are H; and X is H, halo, OR, wherein R is H or C(O)$R^I$, and $R^I$ is $CF_3$, $C_1$-$C_6$ alkyl, $C_5$-$C_{12}$ cycloalkyl, aryl, heteroaryl or $C_1$-$C_6$ alkoxy, or $NR^{II}$,
wherein $NR^{II}$ is a tertiary amine,
with hydroiodic acid, HI, to form the compound of formula I.

**6.** The process of claim 5, wherein the $\Delta$9,11 steroid is a compound of formula Id,

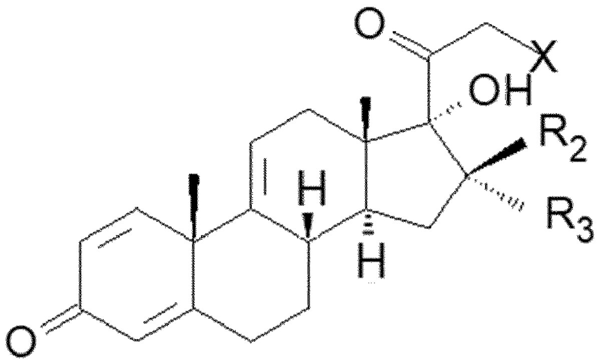

Id

wherein one of $R^2$ or $R^3$ is $CH_3$ and the other is H or both are H; and X is H, halo, OR, wherein R is H or C(O)$R^I$, and $R^I$ is $CF_3$, $C_1$-$C_6$ alkyl, $C_5$-$C_{12}$ cycloalkyl, aryl, heteroarylor $C_1$-$C_6$ alkoxy, or $NR^{II}$, wherein $NR^{II}$ is a tertiary amine, wherein the tertiary amine is part of a linear or cyclic substructure, and the 9,11 epoxy steroid is a compound of formula IId,

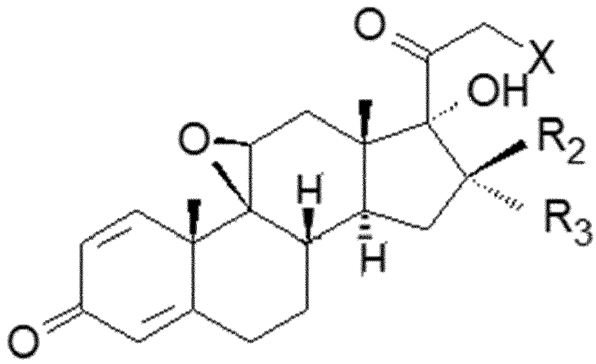

IId

wherein one of $R^2$ or $R^3$ is $CH_3$ and the other is H or both are H; and X is H, halo, OR, wherein R is H or C(O)$R^I$, and $R^I$ is $CF_3$, $C_1$-$C_6$ alkyl, $C_5$-$C_{12}$ cycloalkyl, aryl, heteroaryl or $C_1$-$C_6$ alkoxy, or $NR^{II}$, wherein $NR^{II}$ is a tertiary amine, wherein the tertiary amine is part of a linear or cyclic substructure, with hydroiodic acid, HI, to form the compound of formula Id.

**7.** The process of claim 5 or 6, wherein the Δ9,11 steroid is a compound selected from Vamorolone or Vamorolone Acetate

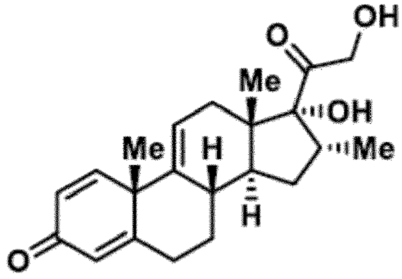

*Vamorolone*

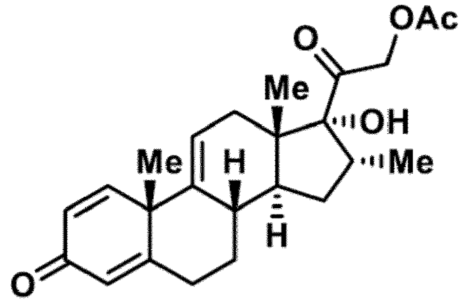

*Vamorolone-acetate*

and the 9,11 epoxy steroid is a compound of 8-DM and 8-DM acetate, respectively.

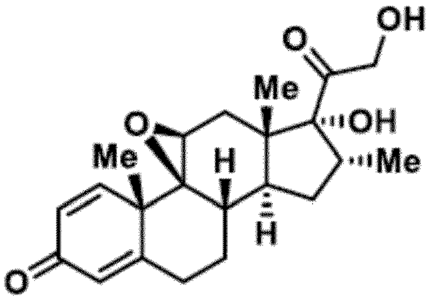

*8-DM*

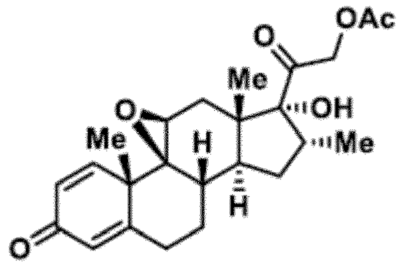

*8-DM-acetate*

**8.** The process of any of claims 5-7, wherein the Δ9,11 steroid is prepared using aqueous HI in an organic solvent and in the presence of an organic acid or without the addition of an organic acid and wherein treatment is carried out at a temperature of below 15°C.

**9.** The process of any of claim 8, wherein the organic solvent is MeCN, $CH_2Cl_2$, or Toluene; or a mixture of two or all three thereof, and
wherein the organic acid is acetic acid.

**10.** The process of any of claims 5-9, wherein the Δ9,11 steroid is prepared using aqueous HI, at a concentration of between 48 and 68 wt%, in toluene.

**11.** The process of any of claims 5-10, wherein if X is OH, the 9, 11 epoxy steroid is acetylated before the treating of the 9,11 epoxy steroid with HI.

**12.** The process of claim 11, wherein the Δ9,11 steroid obtained in claim 15 after treating the 9,11 epoxy steroid with HI, is de-acetylated.

**13.** The process of any of claims 5-12 for preparing a pharmaceutically pure Δ9,11 steroid comprising a step of recrystallization of the crude Δ9,11 steroid obtained in any of claims 2-15, in iPrOH or a mixture of iPrOH and water.

**14.** A process of purifying Vamorolone comprising a step of re-crystallization of crude Vamorolone in isopropanol or in a mixture of water and isopropanol to form purified Vamorolone of 99,5wt% purity or more.

**15.** The compound produced by the process of any of claims 5-14.

**16.** The compound produced by the process of claim 13 or 14.

**17.** A pharmaceutical composition comprising the compound of claim 15 or 16.

Europäisches Patentamt
European Patent Office
Office européen des brevets

# EUROPEAN SEARCH REPORT

Application Number
EP 21 19 1143

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | FR 1 433 301 A (MERCK & CO INC)<br>1 April 1966 (1966-04-01)<br>* example 1 * | 15,16<br>1-13 | INV.<br>C07J71/00<br>C07J5/00<br>A61P5/44<br>A61K31/573 |
| X,D<br>Y | EP 0 969 012 A2 (SCHERING CORP [US])<br>5 January 2000 (2000-01-05)<br>* examples 4, 11 * | 15,16<br>1-13 | |
| Y | CN 108 191 938 A (HUNAN XINHEXIN BIOLOGICAL MEDICINE CO LTD)<br>22 June 2018 (2018-06-22)<br>* page 5, paragraph 25-30 *<br>* examples 1-3 * | 1-13 | |
| Y | GARLASCHELLI L ET AL: "THE DIRECT CONVERSION OF EPOXIDES INTO ALKENES VIA IODOHYDRINS BY IN SITU GENERATED HI",<br>GAZZETTA CHIMICA ITALIANA, SOCIETÀ CHIMICA ITALIANA, IT,<br>vol. 117, no. 5, 1 May 1987 (1987-05-01), pages 251-253, XP000610085,<br>ISSN: 0016-5603<br>* the whole document * | 1-13 | TECHNICAL FIELDS SEARCHED (IPC) |
| Y | FURIASSI LUCIA ET AL: "Limonin as a Starting Point for the Construction of Compounds with High Scaffold Diversity",<br>ANGEWANDTE CHEMIE INTERNATIONAL EDITION,<br>vol. 60, no. 29, 10 June 2021 (2021-06-10), pages 16119-16128, XP055881434,<br>ISSN: 1433-7851, DOI: 10.1002/anie.202104228<br>Retrieved from the Internet:<br>URL:https://onlinelibrary.wiley.com/doi/full-xml/10.1002/anie.202104228><br>* the whole document *<br>-/-- | 1-13 | C07J<br>A61P<br>A61K |

The present search report has been drawn up for all claims

2

| Place of search | Date of completion of the search | Examiner |
|---|---|---|
| The Hague | 9 May 2022 | Watchorn, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 19 1143

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | -& Furiassi Lucia ET AL: "Supporting Information Limonin as a Starting Point for the Construction of Compounds with High Scaffold Diversity Table of Contents", , 12 July 2021 (2021-07-12), XP55881448, Retrieved from the Internet: URL:https://onlinelibrary.wiley.com/action/downloadSupplement?doi=10.1002/anie.202104228&file=anie202104228-sup-0001-misc_information.pdf [retrieved on 2022-01-19] * page 2, scheme S1 * * page 11, scheme S10 * * page 12, scheme S11 * * page 18, scheme S17 * | 1-13 | |
| Y | GEISSMAN T. A. ET AL: "SOME OBSERVATIONS ON THE STRUCTURE OF LIMONIN", THE JOURNAL OF ORGANIC CHEMISTRY, vol. 11, no. 6, 1 November 1946 (1946-11-01), pages 760-770, XP055881452, ISSN: 0022-3263, DOI: 10.1021/jo01176a017 * page 762, last paragraph - page 763, paragraph 1 * | 1-13 | TECHNICAL FIELDS SEARCHED (IPC) |
| X | US 2020/281942 A1 (MCCALL JOHN [US] ET AL) 10 September 2020 (2020-09-10) * claim 1 * | 15-17 | |

-/--

The present search report has been drawn up for all claims

2

| Place of search | Date of completion of the search | Examiner |
|---|---|---|
| The Hague | 9 May 2022 | Watchorn, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 19 1143

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | REEVES ERICA K M ET AL: "VBP15: Preclinical characterization of a novel anti-inflammatory delta 9,11 steroid", BIOORGANIC & MEDICINAL CHEMISTRY, ELSEVIER, AMSTERDAM, NL, vol. 21, no. 8, 18 February 2013 (2013-02-18), pages 2241-2249, XP028596338, ISSN: 0968-0896, DOI: 10.1016/J.BMC.2013.02.009<br>* page 2243, paragraph 2.1 *<br>* abstract * | 14-17 | |
| X | CN 104 974 210 A (TIANJIN JINYAO GROUP CO LTD) 14 October 2015 (2015-10-14)<br>* page 13, paragraphs 76, 77 * | 14-16 | |
| X | CN 102 040 648 A (TIANJIN JINYAO GROUP CO LTD) 4 May 2011 (2011-05-04)<br>* example 5 * | 14-16 | TECHNICAL FIELDS SEARCHED (IPC) |
| X | ZHANG LIQING: "Microbial transformation of 16[alpha]-methyl-5[alpha]-[Delta]9(11)-pregnene-3[beta], 17[alpha], 21-triol-20-one-3[beta], 21-diacetate", YAOXUE XUEBAO, vol. 21, no. 9, 1 January 1986 (1986-01-01), pages 674-679, XP055919039,<br>* page 677, paragraph 4 * | 14-16 | |
| X | US 3 284 477 A (RAUSSER RICHARD C ET AL) 8 November 1966 (1966-11-08)<br>* column 1, paragraph 1 *<br>* example 2 * | 14-17 | |

-/--

The present search report has been drawn up for all claims

2

| Place of search | Date of completion of the search | Examiner |
|---|---|---|
| The Hague | 9 May 2022 | Watchorn, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 19 1143

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ROBINSON C H ET AL: "New Anabolic Agents: 9,11ß-Dihalogenoandrostane Derivatives", JOURNAL OF THE AMERICAN CHEMICAL SOCIETY, vol. 82, no. 17, 1 January 1960 (1960-01-01), pages 4611-4615, XP055919061, Retrieved from the Internet: URL:https://pubs.acs.org/doi/pdf/10.1021/ja01502a042> * page 4613, column 1, last paragraph * ----- | 14-16 | |
| | | | TECHNICAL FIELDS SEARCHED (IPC) |

The present search report has been drawn up for all claims

2

| Place of search | Date of completion of the search | Examiner |
|---|---|---|
| The Hague | 9 May 2022 | Watchorn, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 21 19 1143

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 21 19 1143

The Search Division considers that the present European patent application does not comply with the requirements of unity of invention and relates to several inventions or groups of inventions, namely:

**1. claims: 1-13(completely); 15-17(partially)**

**Use of HI in the conversion of 9,11-steroid epoxides to the corresponding 9(11)-alkene and processes for the conversion of pregnane 9,11-epoxides to the corresponding pregn-9(11)-ene employing HI, as well as products thereof and pharmaceutical compositions of those products.**

---

**2. claims: 14(completely); 15-17(partially)**

**A process for the purification of vamorolone, as well as products thereof and pharmaceutical compositions of those products.**

---

# ANNEX TO THE EUROPEAN SEARCH REPORT ON EUROPEAN PATENT APPLICATION NO.

EP 21 19 1143

This annex lists the patent family members relating to the patent documents cited in the above-mentioned European search report. The members are as contained in the European Patent Office EDP file on
The European Patent Office is in no way liable for these particulars which are merely given for the purpose of information.

09-05-2022

| Patent document cited in search report | | Publication date | Patent family member(s) | | | Publication date |
|---|---|---|---|---|---|---|
| FR 1433301 | A | 01-04-1966 | NONE | | | |
| EP 0969012 | A2 | 05-01-2000 | AT | 188481 | T | 15-01-2000 |
| | | | AT | 395353 | T | 15-05-2008 |
| | | | AT | 397008 | T | 15-06-2008 |
| | | | AU | 696425 | B2 | 10-09-1998 |
| | | | CA | 2191324 | A1 | 07-12-1995 |
| | | | DE | 69514352 | T2 | 24-08-2000 |
| | | | DK | 0063054 | T3 | 19-06-2000 |
| | | | EP | 0763054 | A1 | 19-03-1997 |
| | | | EP | 0969011 | A2 | 05-01-2000 |
| | | | EP | 0969012 | A2 | 05-01-2000 |
| | | | ES | 2140678 | T3 | 01-03-2000 |
| | | | ES | 2306487 | T3 | 01-11-2008 |
| | | | ES | 2306488 | T3 | 01-11-2008 |
| | | | FI | 964739 | A | 28-11-1996 |
| | | | FI | 20050180 | A | 16-02-2005 |
| | | | FI | 20050181 | A | 16-02-2005 |
| | | | GR | 3032714 | T3 | 30-06-2000 |
| | | | HU | 227378 | B1 | 30-05-2011 |
| | | | HU | 227379 | B1 | 30-05-2011 |
| | | | HU | 227380 | B1 | 30-05-2011 |
| | | | IL | 113918 | A | 31-12-1999 |
| | | | IL | 126597 | A | 17-09-2003 |
| | | | IL | 126598 | A | 31-07-2003 |
| | | | JP | 2831472 | B2 | 02-12-1998 |
| | | | JP | H09506113 | A | 17-06-1997 |
| | | | KR | 100226568 | B1 | 15-10-1999 |
| | | | KR | 100290225 | B1 | 15-05-2001 |
| | | | KR | 100290226 | B1 | 15-05-2001 |
| | | | KR | 100290227 | B1 | 15-05-2001 |
| | | | NZ | 287348 | A | 23-12-1998 |
| | | | PT | 763054 | E | 30-06-2000 |
| | | | US | 5502222 | A | 26-03-1996 |
| | | | US | 5616742 | A | 01-04-1997 |
| | | | US | 5750745 | A | 12-05-1998 |
| | | | WO | 9532989 | A1 | 07-12-1995 |
| CN 108191938 | A | 22-06-2018 | NONE | | | |
| US 2020281942 | A1 | 10-09-2020 | NONE | | | |
| CN 104974210 | A | 14-10-2015 | NONE | | | |
| CN 102040648 | A | 04-05-2011 | NONE | | | |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 21 19 1143

This annex lists the patent family members relating to the patent documents cited in the above-mentioned European search report. The members are as contained in the European Patent Office EDP file on
The European Patent Office is in no way liable for these particulars which are merely given for the purpose of information.

09-05-2022

| Patent document cited in search report | Publication date | Patent family member(s) | Publication date |
|---|---|---|---|
| US 3284477 A | 08-11-1966 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5399727 A
- EP 0969012 A


### Non-patent literature cited in the description


- *Bioorganic & Medicinal Chemistry,* 15 April 2013, vol. 21 (8), 2241-2249